**Europäisches Patentamt**

**European Patent Office**

**Office européen des brevets**

(19)

(11) **EP 0 595 276 B1**

(12) **EUROPÄISCHE PATENTSCHRIFT**

(45) Veröffentlichungstag und Bekanntmachung des Hinweises auf die Patenterteilung:
**16.02.2000 Patentblatt 2000/07**

(51) Int. Cl.$^7$: **G01N 11/08**

(21) Anmeldenummer: **93117363.7**

(22) Anmeldetag: **26.10.1993**

(54) **Verfahren und Vorrichtung zur kontinuierlichen Viskositätsmessung**

Method and device for continuous measurement viscosity

Méthode et appareil de mesure de la viscosité en continu

(84) Benannte Vertragsstaaten:
**BE ES FR GB IT NL**

(30) Priorität: **28.10.1992 DE 4236407**

(43) Veröffentlichungstag der Anmeldung:
**04.05.1994 Patentblatt 1994/18**

(73) Patentinhaber:
**GÖTTFERT WERKSTOFF-PRÜFMASCHINEN GMBH**
**D-74722 Buchen (DE)**

(72) Erfinder:
• **Gleissle, Wolfgang**
  **D-76767 Hagenbach (DE)**
• **Schulze, Volkmar**
  **D-74722 Buchen (DE)**

(74) Vertreter:
**Naumann, Ulrich, Dr.-Ing.**
**Patentanwälte,**
**Ullrich & Naumann,**
**Luisenstrasse 14**
**69115 Heidelberg (DE)**

(56) Entgegenhaltungen:
  **EP-A- 0 406 805        DE-A- 2 250 547**
  **DE-A- 4 001 341**

• **DATABASE WPI Week 8547, Derwent Publications Ltd., London, GB; AN 85-295090 & SU-A-1 155 914 (TRILISKII) 15. Dezember 1983**
• **11. STUTTGARTER KUNSTSTOFFKOLLOQUIUM 1989, 1989, STUTTGART Seite 1-17 H.-G FRITZ 'SENSORENTWICKLUNG UND AUTOMATISIERUNGSTENDENZEN IM BEREICH DER KUNSTSTOFFAUFBEREITUNG'**

**Beschreibung**

Die Erfindung betrifft ein Verfahren zur kontinuierlichen oder diskontinuierlichen Messung der Viskosität und deren Verteilung sowie des Schmelzindex MFI bzw. Volumenfließindex MVI von nicht-newtonschen, insbesondere strukturviskosen, viskos-ähnlichen und viskos-unähnhichen Flüssigkeiten, insbesondere von Polymerschmelzen, bei denen die Flüssigkeit mittels einer volumetrischen, regelbaren Dosierpumpe durch einen ersten Kapillarabschnitt und mindestens einen dem ersten Kapillarabschnitt in Serie nachgeschalteten weiteren Kapillarabschnitt geleitet wird, wobei die beiden Kapillarabschnitte unterschiedliche Querschnitte aufweisen,

Insbesondere betrifft die Erfindung Messungen, die sich für die Prozeßkontrolle im Betrieb und im Labor eignen. Derartige Messungen dienen zur schnellen umfassenden und physikalisch eindeutigen Charakterisierung des Fließverhaltens von Flüssigkeiten mit komplexen Fließeigenschaften mit der Möglichkeit zur Interpretation von molekularen Daten, wie z. B. mittleren Molekulargewichten und Molekulargewichtsverteilungen an einer einzigen Probe bei stetiger Messung.

Bekannte kontinuierlich oder diskontinuierlich messende Kapillarviskosimeter oder — rheometer ermitteln die Druckdifferenz zwischen Eingang und Ausgang einer Meßkapillare mit gleichbleibendem Querschnitt bei konstantem Meßvolumenstrom. Aus dem Volumenstrom und der Druckdifferenz läßt sich dann zusammen mit den Abmessungen der Kapillare eine Fließkennzahl, üblicherweise die Viskosität für die Flüssigkeit berechnen. Diese aus einer Messung ermittelte Viskosität ist nur dann die tatsächliche Viskosität dieser Flüssigkeit, wenn deren Fließverhalten durch eine einzige Viskositätszahl gekennzeichnet ist. Solche Flüssigkeiten sind newtonsche Flüssigkeiten. Viele technische Flüssigkeiten (Stoffe, die während Produktionsprozessen als Flüssigkeit auftreten), herausragende Beispiele sind vor allem Polymerschmelzen und Polymerlösungen, sind nicht-newtonsche Flüssigkeiten, deren Viskosität von der Schergeschwindigkeit (bei gegebenen Kapillardimensionen vom Volumenstrom) selbst abhängen. Zur Beschreibung deren Fließverhaltens sind Viskositäts- oder andere Fließfunktionen z.B. die Schubspannungsfunktion notwendig.

Zur Ermittlung einer Fließfunktion oder Abschnitte einer Fließfunktion müssen mit dem Viskosimeter oder Kapillarrheometer zeitlich nacheinander aus unterschiedlichen Volumenströmen und dazugehörigen Druckdifferenzen Wertepaare ermittelt werden, um daraus Punkte der Viskositäts- bzw. einer anderen Fließfunktion zu ermitteln. Die Messung von Fließbereichen durch einzelne Punkte erlaubt dann erst über entsprechende Korrekturverfahren die Berechnung von wahren Viskositäts- bzw. Fließkurven. Die Messung von nur einer Druckdifferenz bei nur einem Volumenstrom ergibt bei nicht-newtonschen Flüssigkeiten nur eine sogenannte „scheinbare" Viskosität, da die Ermittlung der entsprechenden Fließkennzahlen nur über die newtonschen Fließgesetze möglich ist. Wird ein solches Einpunktmeßverfahren in einem Produktionsprozeß zur Kontrolle eingesetzt, dann können z. B. Änderungen des Meßsignales nicht direkt in die richtigen tatsächlichen Änderungen der Viskosität umgerechnet werden, es sei denn, das Viskosimeter wurde vorher mit einer entsprechenden Testflüssigkeit kalibriert. So kalibrierte Viskosimeter sind dann aber nur für die einzige Flüssigkeit als wahre Viskosimeter verwendbar. Für andere Flüssigkeiten müssen diese Viskosimeter über den ganzen Meßbereich neu kalibriert werden. Will man jedoch einen Fließbereich einer Flüssigkeit meßtechnisch erfassen, um Abweichungen vom newtonschen Fließverhalten feststellen zu können, müssen mit solchen Viskosimetern mit einer einzigen Kapillare zeitlich nacheinander mehrere Versuche bei unterschiedlichen Schergeschwindigkeiten (d. h. mit unterschiedlichen Volumenströmen) durchgeführt werden. Einerseits führt dies zu einer längeren Meßzeit. Andererseits sind die Messungen bei verschiedenen Schergeschwindigkeiten an verschiedenen Flüssigkeitsproben mit einem Kapillarviskosimeter vorzunehmen.

Um wenigstens einen begrenzten Fließbereich ohne Änderung des Betriebszustandes eines z. B. Kontroll-(Prozeß-)viskosimeters zu erfassen, wurde schon mehrfach vorgeschlagen zwei oder mehrere Kapillaren mit unterschiedlichen Abmessungen hintereinanderzuschalten, über deren Länge jeweils der Druckabfall gemessen wird, oder Kapillaren mit längs des Strömungsweges sich z. B. keilförmig änderndem Querschnitt zu verwenden, bei denen der Druckabfall abschnittsweise gemessen wird, (siehe M. Heckenbach, E. Muschelknautz, Viskoelastische Betriebsmessungen, Vortragskurzfassung der Jahrestagung der Deutschen Rheologischen Gesellschaft (DRG), Aachen, 1979; H. G. Fritz, Sensorentwicklung und Automatisierungstendenzen im Bereich der Kunststoffaufbereitung, 11. Stuttgarter Kunststoffkolloquium, 1989; A. Pabedinskas, W.R. Cluett, S.T. Balke in Polymer Engineering and Science, Mid-March 1991, Vol. 31, No. 5, Seiten 365-375) und U.S. Patentschrift 4, 425,790). Alle diese Kapillaranordnungen werden dann bei einem oder mehreren zeitlich konstanten Volumenströmen betrieben. Aus den über die einzelnen Kapillaren oder Kapillarabschnitten (Keildüse) gemessenen Druckdifferenzen können dann zwei oder mehrere Punkte einer Viskositätsfunktion ermittelt werden. Dabei erfolgen die Messungen der zwei Viskositäten gleichzeitig und an derselben Probe, was gerade bei Kontrollmessungen gegenüber der vorher beschriebenen seriellen Methode einen wesentlichen Vorteil bietet. Bei zwei Kapillaren läßt sich dann auch für einen mittleren Wert zwischen den Meßpunkten ein Korrekturwert zur Berechnung der wahren Viskosität angeben. (Dies geschieht am besten dadurch, daß das Fließverhalten durch ein einfaches Fließgesetz, wie das von Ostwald de Waele, das auch Potenzgesetz genannt wird, angenähert wird). Eine einfache Formulierung enthält Gl. 1, wobei die Schubspannung $\tau$ mit der Schergeschwindigkeit $\dot{\gamma}$ über einen Fließexponenten n und eine Konsistenz k verbunden ist.

$$\tau\,(\dot{\gamma}) = k\,\dot{\gamma}^{\,n} \tag{1}$$

Die dieser Schubspannungsfunktion entsprechende Viskositätsfunktion ist in Gl. (2) dargestellt, wobei deren Fließexponent m direkt mit dem Exponenten n über Gl. (3) verknüpft ist.

$$\eta(\dot{\gamma}) = \frac{\tau\,(\dot{\gamma})}{\text{------}} = k\,\dot{\gamma}^{(n-1)} = k\,\dot{\gamma}^{m} \tag{2}$$

$$m = n - 1 \tag{3}$$

Zwei oder mehrere Meßpunkte erlauben dann die Berechnung eines oder mehrerer wahrer Viskositätswerte und zeigen an, wie stark sich die Viskosität bei Änderung der Schergeschwindigkeit ändert. Die Stärke der Änderung der Viskosität bzw. der Schubspannung mit der Schergeschwindigkeit wird üblicherweise mit einem Fließexponenten n beschrieben.

$$n = d\,\log\,\tau\,/\,d\log\,(\dot{\gamma}) \tag{4}$$

$$= \frac{\log\,(\Delta\,p_1/\Delta\,p_2)}{\log\,(\dot{\gamma}_1\,/\,\dot{\gamma}_2)} \tag{5}$$

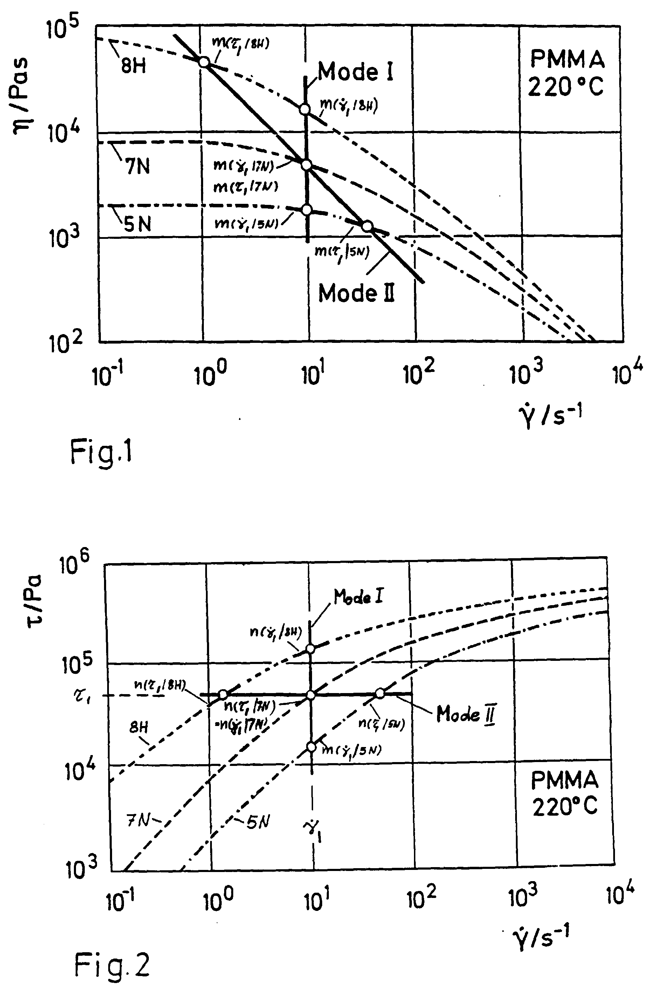

Dieser Fließexponent hat den Wert 1 bei newtonschen Flüssigkeiten, deren Viskosität konstant ist ($\tau$ ändert sich proportional zu $\dot{\gamma}$) oder ist ungleich 1 für nicht-newtonsche Flüssigkeiten. Normalerweise kann die Fließfunktion einer realen nicht-newtonschen Flüssigkeiten nur bereichsweise mit einem konstanten Fließexponenten n beschrieben werden. Für sich stetig mit $\dot{\gamma}$ ändernde Viskositäten ist n nach Gl. (4) die Steigung der Schubspannungsfunktion in einem logarithmischen Koordinatennetz log $\tau$ - log $\dot{\gamma}$ (siehe dazu Fig. 2). Bei polymeren Flüssigkeiten, wie z. B. Polymerschmelzen und Polymerlösungen nimmt die Viskosität mit steigender Schergeschwindigkeit ab. Dies sind sogenannte strukturviskose Flüssigkeiten. Für solche Flüssigkeiten ist der Fließexponent n kleiner als 1. Je stärker sich der Fließexponent n von 1 unterscheidet, desto stärker ändert sich die Viskosität mit steigender Schergeschwindigkeit. Der Fließexponent n ist also ein Maß für die Strukturviskosität oder wenn man die Viskositätsfunktion $\eta(\dot{\gamma})$ als Viskositätsverteilung betrachtet, ist n (bzw. m = n - 1) ein Maß für die Verteilungsbreite oder das Spektrum der Viskosität.

Mit einem Viskosimeter, das nun mit zwei oder mehr Meßkapillaren oder mit einer im Querschnitt veränderlichen Meßkapillare ausgerüstet ist, kann nun bei konstantem Volumenstrom neben der wahren Viskosität auch der Fließexponent n an einer oder mehreren Stellen ermittelt werden. Eine solche Messung ergibt also neben der Viskosität auch ein Maß für die Verteilungsbreite bzw. das Spektrum der Viskosität.

Ist ein solches Viskosimeter in der Prozeßkontrolle eingesetzt, dann zeigen Änderungen der Druckdifferenzen über die einzelnen Kapillaren unterschiedlicher Abmessungen bzw. Kapillarabschnitte in einer Keilkapillare, über die jeweils derselbe konstante Volumenstrom fließt, eine sich verändernde Viskosität und der Vergleich der veränderten Druckabfälle über den beiden Kapillaren oder Kapillarabschnitten die Änderung des Fließexponenten an. Dieses Verfahren liefert bei Flüssigkeiten mit Fließfunktion (also nicht newtonsch), z. B. strukturviskosen Polymerschmelzen, bei der Änderung der Viskosität jedoch immer auch gleichzeitig eine Änderung des Fließexponenten, also scheinbar eine andere Viskositätsverteilung, obwohl sich die gesamte Viskositätsverteilung häufig eigentlich nicht ändert.

Fig. 1 zeigt die Viskositätsfunktion und Fig. 2 die Schubspannungsfunktion von 3 unterschiedlichen Polymethylmetacrylaten (PMMA) mit ähnlicher Molekulargewichtsverteilung. Auf der Abszisse ist jeweils die Schergeschwindigkeit $\dot{\gamma}$ und auf der Ordinate die Viskosität $\eta$ (Fig. 1) bzw. die Schubspannung $\tau$ (Fig. 2) angegeben. Die Steigungen an den herausgegriffenen Meßpunkten sind durch Geradenstücken angedeutet; sie entsprechen den Fließexponenten m der Viskositätsfunktion in Fig. 1 und den Fließexponenten n der Schubspannungsfunktion in Fig. 2. Die Gerade Mode I gilt für die Messung bei konstanter Schergeschwindigkeit $\dot{\gamma}_1 = 10\ s^{-1}$ und die Gerade Mode II gilt für die Messung bei konstanter Schubspannung $\tau_1 = 5 \cdot 10^4$ Pa. Die den Meßpunkten in den Fig. 1 und 2 zugehörigen Meßwerte sind in der Tabelle wiedergegeben. Die Spalten 3 bis 5 in der Tabelle (siehe letzte Seite) unterstreichen, daß bei Messungen mit konstanter Schergeschwindigkeit (im Bereich Mode I) sowohl unterschiedliche Viskositäten als auch unterschiedliche Fließexponenten m und n ermittelt werden.

Die Viskosität von Flüssigkeiten ist neben vielen anderen Parametern von der Temperatur abhängig, häufig sogar sehr stark. Widersprüchliche Ergebnisse ergeben sich nun bei Anwendung des oben beschriebenen Meßverfahrens, wenn zwar die zu testende Flüssigkeit dieselbe bleibt, die Temperatur der Flüssigkeit sich jedoch ändert. Entsprechend der Temperaturänderung wird mit einer Mehrkapillarenanordnung bei konstantem Volumenstrom nun eine entsprechend geänderte Viskosität ermittelt, jedoch wird beim Vergleich der einzelnen Kapillaren auch ein neuer Fließexponent aus den Meßwerten berechnet, obwohl sich bei einer Temperaturänderung die Breite des Viskositätsspektrums, das auf molekularen Daten beruht, nicht ändert. Dies führt zwangsläufig zu Fehlinterpretationen bei der Beurteilung von Meßdaten. Die Messung von Fließeigenschaften, z. B. zu Kontrollzwecken, bei konstantem Volumenstrom, der durch Kapillaren mit unterschiedlichen Abmessungen geführt wird, liefert zwar eine wahre Viskosität $\eta$ und mit m ein Maß für die Änderung der Viskosität mit der Schergeschwindigkeit, so daß mehr Informationen über das Fließverhalten vorliegt, beide sind jedoch nicht eindeutig zueinander korrelierbar (was man am Beispiel der reinen Temperaturänderung unmittelbar erkennen kann). Es können z. B. daraus keine unmittelbaren qualitativen und quantitativen Aussagen über molekulare Änderungen wie mittleres Molekulargewicht und Molekulargewichtsverteilung in der Flüssigkeit gemacht werden.

Polymerschmelzen und Polymerlösungen sind strukturviskose Flüssigkeiten und haben bei kleinen Schergeschwindigkeiten ihre höchste Viskosität. Die Viskosität ist dort auch unabhängig von $\dot{\gamma}$. Man nennt diesen Bereich den unteren newtonschen Fließbereich und die dazugehörige Viskosität die Nullviskosität $\eta_O$. Diese Nullviskosität ist direkt mit dem mittleren Molekulargewicht $M_w$ der Flüssigkeit gekoppelt, sh. M. Pahl, W. Gleißle, H. M. Laun, Praktische Rheologie der Kunststoffe und Elastomere, VDI-Verlag GmbH, 1991, S. 136. Aus der Änderung von $\eta_O$, kann die Änderung des mittleren Molekulargewichtes ermittelt werden. Jedoch ist $\eta_O$ und damit $M_w$ häufig nur bei sehr extrem kleinen $\dot{\gamma}$ meßbar, die von Viskosimetern nicht erreichbar bzw. wegen der sehr langen Meßzeiten (manchmal Stunden) bei Kontrollmessungen nicht praktikabel sind.

Die Strukturviskosität, d. h. die Art der Abnahme der Viskosität mit steigender Schergeschwindigkeit, ist dann von der Molekulargewichtsverteilung abhängig (z. B. M. Pahl, W. Gleißle, H. M. Laun, Praktische Rheologie der Kunststoffe und Elastomere, VDI-Verlag GmbH, 1991, S. 136). Flüssigkeiten mit ähnlichen Molekulargewichtsverteilungen ergeben auch ähnliche Viskositätsfunktionen. Man nennt diese Flüssigkeiten viskos ähnlich (W. Gleißle, Speed- or Stress-controlled Rheometry, in A. A. Collyer und L.A. Utracki, Polymer Rheology and Processing, Elsevier Applied Science, Publishers Ltd., London, New York, 1990, S. 361).

Nutzt man diese Eigenschaft der viskosen Ähnlichkeit, so kann mit einem z. B. Kontrollviskosimeter, das wie üblich mit nur einer Kapillare nicht bei konstanter Schergeschwindigkeit sondern bei konstanter Druckdifferenz arbeitet, auch aus Messungen bei höheren Schergeschwindigkeiten die Nullviskosität ermittelt werden, wie dies in der europäischen Patentschrift 406 805, die der U.S. Patentschrift 5, 172,585 (W. Gleissle) entspricht, und in der U.S. Patentschrift 4, 817,416 (J.F. Blanch et al) beschrieben ist. Dabei ist aber strenge Voraussetzung, daß die viskose Ähnlichkeit erhalten bleibt, wenn sich die Viskosität ändert (siehe dazu Spalte 10 in Tab. 1). Eine weitere Fließkennzahl, die bei konstanter Druckdifferenz ermittelt wird, ist der weltweit standardisierte Schmelzindex MFI oder der Volumenfließindex MVI. Der Schmelzindex MFI ist das pro 10 min durch eine Standarddüse extrudierte Gewicht einer Flüssigkeitsmenge und der Volumenindex MVI die entsprechende Flüssigkeitsmenge in ccm bei vorgegebener Temperatur und Druckdifferenz.

In der EP-A-0 406 805 werden Echtzeit-Kapillarrheometer-Anordnung zur kontinuierlichen und diskontinuierlichen Messung der Viskosität von viskos-elastischen Flüssigkeiten beschrieben, die allesamt lediglich eine Meßkapillare umfassen, welche über ihre gesamte Länge eine gleichbleibende Querschnittsform und Querschnittsfläche aufweist.

Entsprechend der Druckschrift Database WPI, Week 8547, Derwent Publications Ltd., London, GB, AN: 85-295090 & SU-A-1155914 (Triliskii), 15. Dezember 1983 werden die rheologischen Eigenschaften von Schmelzen bestimmt, indem die Meßproben durch zwei in Serie geschaltete Kapillaren geleitet werden. Aus der Zeichnung ergibt sich, daß diese beiden Kapillaren unterschiedliche Querschnitte aufweisen können. Der Druckabfall über einer der beiden Kapillare wird konstant gehalten. Anhand des sich daraufhin einstellenden Druckabfalls über der anderen Kapillare werden dann die rheologischen Eigenschaften der Meßprobe bestimmt. Die hier verwendete Vorrichtung umfaßt ausdrücklich keine Dosierpumpe zur Regelung des Druckabfalls über der einen Kapillare. Zur Regelung des Druckabfalls wird hier ein Regelventil verwendet. Des weiteren wird hier lediglich der Druckabfall über einer der beiden Kapillaren festgehalten und nicht über beiden in Serie geschalteten Meßkapillaren. Außerdem wird entsprechend dem bekannten Verfahren der sich aufgrund des vorgegebenen Druckabfalls über der einen Kapillare einstellende Druckabfall in der anderen Kapillaren gemessen und nicht die sich aufgrund der Druckabfälle in den Kapillaren einstellenden Volumenströme und die daraus resultierenden Schergeschwindigkeiten. Demnach kann mit dem bekannten Verfahren auch nicht der Fließexponent n bestimmt werden. Schließlich sei noch darauf hingewiesen, daß mit dem bekannten Verfahren lediglich das Verhältnis der Viskositäten $\eta_1$ und $\eta_2$ in den beiden in Serie geschalteten Kapillaren ermittelt wird und nicht die Viskositätsfunktion selbst.

In der DE-A-2 250 547 wird eine Vorrichtung zur kontinuierlichen Messung der Viskosität oder Konsistenz oder Stoffdichte einer strömenden Flüssigkeit beschrieben, die ein sich erweiterndes Durchflußrohr als Meßgeber sowie zwei im Abstand voneinander angeordnete Meßstellen zur Messung des Differenzdruckes der durchströmenden Flüssigkeit umfaßt, wobei die eine Meßstelle im Bereich geringeren und die andere Meßstelle im Bereich größeren Rohrquerschnitts liegt und die zwischen beiden Meßstellen liegende Erweiterung den durch die Strömungsgeschwindigkeit bedingten Differenzdruck kompensiert.

In der Druckschrift 11. Stuttgarter Kunststoffkolloquium 1989, Stuttgart, Seite 1-17; H.-G. Fritz: „Sensorentwicklung und Automatisierungstendenzen im Bereich der Kunststoffaufbereitung" wird ein Schlitzkapillarrheometer beschrieben, das als On-Line-Rheometer eingesetzt wird. Die Kunststoffschmelze, deren rheologische Eigenschaften bestimmt werden sollen, wird mit Hilfe einer Zahnradpumpe durch die Kapillarenanordnung geleitet. Durch Variation der Drehzahl der Zahnradpumpe werden definierte Volumenströme eingestellt, für die dann jeweils der Druckgradient, d.h. der Druckabfall, über einzelnen Abschnitten der Kapillarenanordnung gemessen wird. Auf diese Weise lassen sich die wahre Fließkurve $\gamma(\tau)$ und die Viskositätsfunktion $\eta(\gamma)$ ermitteln. Bild 6 dieser Druckschrift zeigt ein Schlitzkapillarrheometer, bei dem zwei unterschiedlich dimensionierte Flachschlitzkapillaren in Reihe geschaltet sind. In diesem Zusammenhang wird ausgeführt, daß ein derartiges Konzept bei konstanter Zahnradpumpendrehzahl die simultane Ermittlung zweier <$\dot{\gamma}$, $\tau$>-Wertepaare erlaubt, als eine mit dem Quotienten $MFI_{10}/MFI_{216}$ korrespondierende Größe. Gleichzeitig ergeben sich daraus deutlich kürzere Meßzeiten bei der Ermittlung vollständiger Fließkurven und Viskositätsfunktionen, weil die Zahl der anzufahrenden Pumpendrehzahlen verringert wird und jede Drehzahlvariation ein Übergangsverhalten des Meßsystems auslöst. Entsprechend dem bekannten Verfahren können also aus den über den einzelnen Kapillarabschnitten gemessenen Druckdifferenzen zwei oder auch mehrere Punkte einer Viskositätsfunktion ermittelt werden. Über die Beziehung

$$\eta(\dot{\gamma})=k\dot{\gamma}^{(n-1)}=k\dot{\gamma}^{m} \ ,$$

läßt sich die Stärke der Änderung der Viskosität mit der Schergeschwindigkeit $\dot{\gamma}$ ermitteln, wobei k eine Konsistenz ist. Der Fließexponent n ergibt sich als

$$n = \frac{\log (\Delta p_1 / \Delta p_2)}{\log (\dot{\gamma}_1 / \dot{\gamma}_2)} \ ,$$

wobei $\Delta p_1$ und $\Delta p_2$ die Druckabfälle über den Kapillarabschnitten 1 und 2 sind und $\dot{\gamma}_1$, $\dot{\gamma}_2$ die sich in diesen Kapillarabschnitten einstellenden Schergeschwindigkeiten.

Der Fließexponent n ist ein Maß für die Strukturviskosität oder, wenn man die Viskositätsfunktion $\eta(\dot{\gamma})$ als Viskositätsverteilung betrachtet, ein Maß für die Verteilungsbreite oder das Spektrum der Viskosität.

Normalerweise kann die Fließfunktion einer realen, nicht-newtonschen Flüssigkeit nur bereichsweise mit einem konstanten Fließexponenten n beschrieben werden.

Ein bei Anwendung des bekannten Verfahrens auftretendes Problem liegt darin, daß es für nicht-newtonsche Flüssigkeiten, z.B. strukturviskosen Polymerschmelzen, bei Änderungen der Viskosität auch immer eine Änderung des Fließexponenten liefert, also scheinbar eine andere Viskositätsverteilung, obwohl sich die gesamte Viskositätsverteilung häufig eigentlich gar nicht ändert.

Das bekannte Verfahren zur Bestimmung von rheologischen Eigenschaften einer Kunststoffschmelze erweist sich außerdem auch als problematisch, wenn zwar die zu testende Schmelze dieselbe bleibt, sich aber die Temperatur dieser Schmelze ändert. Die Viskosität von Flüssigkeiten ist nämlich - häufig sogar sehr stark - von der Temperatur abhängig. Wird die Kapillaranordnung nämlich - entsprechend dem bekannten Verfahren - mit einem konstanten Volumenstrom beschickt, so werden für unterschiedliche Temperaturen neben unterschiedlichen Viskositäten auch unterschiedliche Fließexponenten ermittelt, obwohl sich bei einer Temperaturänderung die Breite des Viskositätsspektrums, das auf molekularen Daten beruht, nicht ändert. Dies führt zwangsläufig zu Fehlinterpretationen bei der Beurteilung der mit dem bekannten Verfahren gewonnenen Meßdaten.

Im Ergebnis liefert das bekannte Verfahren zwar eine wahre Viskosität $\eta$ und ein Maß für die Änderung der Viskosität mit der Schergeschwindigkeit. Diese rheologischen Eigenschaften sind jedoch nicht eindeutig zueinander korrelierbar, was man am Beispiel der reinen Temperaturänderung unmittelbar erkennen kann. So können mit Hilfe des bekannten Verfahrens keine unmittelbaren qualitativen und quantitativen Aussagen über molekulare Änderungen, wie mittleres Molekulargewicht und Molekulargewichtsverteilung, in der Meßprobe gemacht werden.

Ausgehend von diesem Stand der Technik liegt der Erfindung die Aufgabe zugrunde, ein Meßverfahren und eine Kapillarenanordnung zu schaffen, bei denen die gemessenen Größen, wie Viskosität und Fließexponent, korrelierbar sind.

Das erfindungsgemäße Verfahren löst die voranstehende Aufgabe durch die Merkmale des Patentanspruchs 1. Danach wird der durch die in Serie geschalteten Kapillarabschnitte geleitete Volumenstrom derart geregelt, daß der Druckabfall längs wenigstens zweier Kapillarabschnitte konstant gehalten wird. Der von der Pumpe abgegebene Volumenstrom wird bestimmt. Aus dem Volumenstrom werden unter Berücksichtigung der Geometrie der Kapillarabschnitte die Schergeschwindigkeiten $\dot{\gamma}_1$ und $\dot{\gamma}_2$ in den Kapillarabschnitten bestimmt. Als Maß für die Strukturviskosität bzw. die Breite der Viskositätsverteilung der Flüssigkeit wird ein Fließexponent n bestimmt als

$$n = \frac{\log (\Delta p_1 / \Delta p_2)}{\log (\dot{\gamma}_1 / \dot{\gamma}_2)} \quad ,$$

wobei $\Delta p_1$ und $\Delta p_2$ die Druckabfälle über den Kapillarabschnitten sind, und für jede Schergeschwindigkeit $\dot{\gamma}_1$, $\dot{\gamma}_2$ wird die Viskosität bestimmt als

$$\eta(\dot{\gamma}) = k\dot{\gamma}^{(n-1)} = k\dot{\gamma}^m \quad ,$$

wobei k eine Konsistenz ist.

Wird eine serielle Anordnung von zwei Kapillaren, wie vorher beschrieben, nicht, wie in der Fach- und Patentliteratur angegeben und empfohlen, bei konstantem Volumenstrom sondern bei konstanter Druckdifferenz längs einer Kapillare bzw. einem Kapillarenabschnitt betrieben, so können die Aussagen aus der viskosen Ähnlichkeit so weitgehend genutzt werden, daß neben der Änderung einer Bezugsviskosität oder der Nullviskosität im gegebenen Fall auch quantitativ die Änderung der Viskositätsverteilung bzw. die Änderung der Molekulargewichtsverteilung ermittelt werden kann.

Das Ziel wird erfindungsgemäß nur mit einer Kapillarenanordnung mit zwei oder mehreren hintereinander geschalteten Kapillaren mit unterschiedlichem Querschnitt oder einer Kapillare mit längs der Strömungsrichtung sich änderndem Querschnitt erreicht, die so betrieben wird, daß über eine Kapillare oder einen Kapillarabschnitt oder die gesamte Kapillaranordnung eine konstante Druckdifferenz eingehalten wird.

Zwei viskos ähnliche Flüssigkeiten (das können z. B. Polymerschmelzen mit unterschiedlichem Molekulargewicht, jedoch ähnlicher Molekulargewichtsverteilung sein), ergeben in einem Kapillarrheometer mit mehreren Kapillaren oder einer Kapillaren mit längs der Längsachse veränderlichem Querschnitt bei Messungen mit konstant gehaltener Druckdifferenz unterschiedliche Volumenströme, aus denen sich dann bei gleichen Druckdifferenzen unterschiedliche Viskositäten berechnen. Wegen der viskosen Ähnlichkeit bleibt jedoch der z. B. nach Gl. (4) berechnete Fließexponent n konstant. (Siehe dazu die Fig. 1 und Fig. 2 und die Tabelle bei Mode II, Spalten 7 und 8). Das ist ein fundamentaler Unterschied zu den Messungen mit konstantem Volumenstrom. Dort ergeben Messungen an viskos ähnlichen Fluiden aber unterschiedlichem Molekulargewicht sowohl unterschiedliche Viskositäten als auch unterschiedliche Fließexponenten, die nicht unmittelbar korreliert werden können (Spalten 3 und 4 in der Tabelle; Mode I).

Messungen bei konstant gehaltener Druckdifferenz (physikalisch: konstante Schubspannung), die unterschiedliche Viskositäten aber gleichen Fließexponenten ergeben, zeigen viskos ähnliche Flüssigkeiten an. Es ändert sich zwar die für diese Druckdifferenz (Schubspannung) ermittelte Viskosität, die Viskositätsverteilung, d. h. die Viskositätsfunktion ist jedoch ähnlich (was einen gleichen Kurvenzug in einem Viskositätsdiagramm entspricht). Bezogen auf molekulare Größen läßt sich aus diesem Meßergebnis unmittelbar schließen, daß sich das mittlere Molekulargewicht geändert hat, die Molekulargewichtsverteilung aber ähnlich ist (gleiches Verhältnis von kleinen zu großen Molekülen). Für diese Anordnung und diese Betriebsart sind Viskositäten und Fließexponent mit molekularen Daten und Bezugsgrößen eindeutig korrelierbar.

Für eine Kapillarrheometer, das so zur Ermittlung der Nullviskosität betrieben wird, bedeutet Konstanz der Fließexponenten auch eine korrekte Ermittlung der Nullviskosität oder des mittleren Molekulargewichtes, da die Bedingung der viskosen Ähnlichkeit kontrolliert erfüllt ist.

Dies kann unmittelbar aus Spalte 10 der Tabelle ersehen werden, da alle bei Mode II (konstante Schubspannung) ermittelten Viskositäten $\eta$ $(\tau_1)$ sich proportional zu den entsprechenden Nullviskositäten $\eta_O$ ändern. Vergleicht man dies mit den Ergebnissen von Mode I (konstante Schergeschwindigkeit), so ergibt sich dort, daß $\eta_O/\eta$ $(\dot{\gamma}_1)$ sich

trotz viskoser Ähnlichkeit von Type zu Type ändert.

Wird die zu testende Flüssigkeit einer reinen Temperaturerhöhung unterworfen, so wird dies bei dem erfindungsgemäßen Verfahren und Vorrichtungen durch eine der Viskositätsänderung proportionale Drehzahländerung der Dosierpumpe angezeigt. In diesem Fall bleibt das gemessene Verhältnis der Druckabfälle über den einzelnen Kapillaren bzw. Kapillarabschnitten in jedem Fall konstant. Das Verhältnis der durch die Temperaturänderung hervorgerufenen neuen Drehzahl zur Drehzahl bei der vorhergegangenen Temperatur wird als Temperaturshiftfaktor a bezeichnet. Mit diesem Verfahren und dieser Vorrichtung können Temperaturshiftfaktoren unmittelbar und kontrolliert (n = konst) ermittelt werden. Werden beim Vergleich von zwei Flüssigkeiten mit einem erfindungsgemäßen Kapillarrheometer mit mehreren Kapillaren oder Kapillaren mit veränderlichem Querschnitt, die bei konstanter Druckdifferenz betrieben wird, sowohl unterschiedliche Viskositäten als auch unterschiedliche Fließexponenten ermittelt, so sind diese Flüssigkeiten viskos unähnlich. Sie haben unterschiedliche Molekulargewichte und unterschiedliche Molekulargewichtsverteilungen. Für eine solche Anordnung, die aus Daten bei höheren $\dot{\gamma}$ die Nullviskosität angeben soll, bedeutet ungleicher Fließexponent dann eine falsche Berechnung von $\eta_O$. Eine solche Meßführung ergibt so ein sich selbst kontrollierendes Meßverfahren.

Messungen mit derselben Anordnung von mehreren Kapillaren, betrieben bei konstantem Volumenstrom, ergeben zwar auch unterschiedliche Viskositäten und unterschiedliche Fließexponenten, jedoch läßt sich kein qualitativer und quantitativer Rückschluß auf die Änderungen der mittleren Molekulargewichte und deren Verteilung ziehen.

Rheometer, mit denen erfindungsgemäß Viskositäten und Fließexponenten, die eindeutig korrelierbar sind, gleichzeitig ermittelt (simultan) werden können, weisen entweder eine aus der Hintereinanderschaltung von mindestens zwei oder mehr Strömungskanälen (Kapillaren) mit unterschiedlichen Querschnittsabmessungen, über die dann jeweils der Druckabfall mittels Druckgeber ermittelt wird, oder einen Strömungskanal (Kapillare) auf, dessen Querschnittsabmessung sich längs des Strömungsweges stetig ändert und in dem abschnittsweise der Druckabfall ermittelt wird. Dabei können die einzuhaltenden Druckdifferenzen $\Delta p$ z. B. bei $\Delta p = 0,5$ bar bis $\Delta p = 500$ bar liegen. Die Querschnittsform der Kapillare kann kreisförmig oder rechteckig sein, wobei bei den Kapillaren mit stetig ändernden Querschnitten sich kegelförmige bzw. keilförmige Kapillaren ergeben. Diese Kapillaranordnungen werden mittels einer geregelten Dosierpumpe, die für Polymerschmelzen und —lösungen in der Regel eine Zahnradpumpe ist, mit einem Volumenstrom versorgt, der über alle hintereinandergeschalteten (seriell angeordneten) Kapillaren gleich groß ist. Dieser Volumenstrom wird nun über die Drehzahl der Dosierpumpe erfindungsgemäß so geregelt, daß der Druckabfall über eine Kapillare oder bei den keil— bzw. kegelförmigen Kapillaren über einen Kapillarenabschnitt oder über die gesamte Kapillaranordnung konstant gehalten wird. Die Dosierpumpe wird also nicht bei konstanter Drehzahl bzw. konstantem Volumenstrom — wie bisher — betrieben, sondern hat eine zusätzliche oder andere Regeleinrichtung, die die Dosierpumpe derart regelt, daß über die ganze oder zumindest einen Teil der Kapillaranordnung ein konstanter Druckabfall herrscht. Eine Änderung der Viskosität (bzw. des mittleren Molekulargewichtes) wird dann durch eine Änderung der Pumpendrehzahl und eine Änderung der Viskositätsverteilung durch eine Änderung des Verhältnisses der Druckabfälle über die einzelnen Kapillaren bzw. einzelnen Kapillarabschnitte angezeigt. (Mit den Gl. (3) und (4) können dann die entsprechenden Fließexponenten m und n ermittelt werden). Ändert sich eine Flüssigkeit viskos ähnlich (d. h. auch molekular ähnlich), dann bleiben die Verhältnisse der Druckabfälle konstant. (Die die Kapillarenanordnung verlassende Flüssigkeit kann ins Freie abgeführt werden, oder wenn sie einem Prozeß entnommen wurde, in den Prozeß wieder zurückgeführt oder ebenfalls ins Freie abgeführt werden). Im Falle von Flüssigkeiten, deren Viskosität vom Druck selbst abhängt, ist es dann oft sinnvoll, die Kapillarenanordnung durch Zusatzeinrichtungen, z. B. eine zusätzliche Pumpe am Ausgang der Kapillarenanordnung gegen Druckschwankungen im Prozeß zu schützen bzw. gezielt einen definierten Druck einzustellen mit einer Pumpe, die mit einer zusätzlichen Regeleinrichtung versehen ist. Damit kann die Viskositätsmessung auf Prozeßbedingungen eingestellt werden. Die Ausführungsbeispiele zeigen nur Anordnungen mit zwei Kapillaren bzw. zwei Meßabschnitten mit einer Keil— und Kegelkapillare. Eine Zusatzpumpe 2 kann auch so geregelt werden, daß am Ausgang der Kapillarenanordnung ein konstanter Druck vorliegt.

Ausführungsbeispiele sind anhand einer Zeichnung näher erläutert, in der zeigt:

Fig. 3: ein Rheometer mit einer sich kreiskegelförmig verjüngenden Kapillare, über deren Länge zwei Kapillarabschnitte durch Druckmeßwertgeber definiert sind (nicht erfindungsgemäß),

Fig. 4: ein dem Rheometer nach Fig. 3 ähnliches Rheometer, jedoch mit einer sich verjüngenden Schlitzkapillare (nicht erfindungsgemäß),

Fig. 5: ein Rheometer mit zwei Kapillarabschnitten unterschiedlichen, oben jeweils gleichen rechteckigen Querschnitts, die durch einen konischen Übergang in Serie miteinander verbunden sind, und

Fig. 6: ein Rheometer mit zwei in Serie angeordneten Kapillaren unterschiedlich, aber jeweils gleichen runden Querschnitts mit sprunghaftem Übergang, bei dem Druckmeßgeber zu beiden Seiten der ersten und zweiten Kapillare vorgesehen sind.

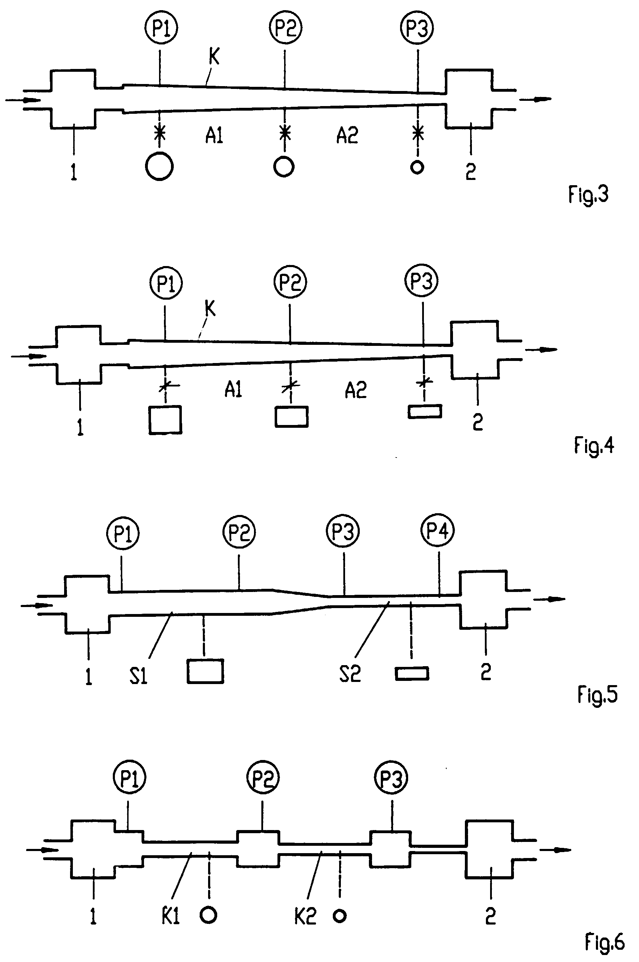

Die in Fig. 3 dargestellte Ausführungsform eines Kapillarrheometers besteht aus einer Dosierpumpe1, die einer Kapillare K mit sich stetig verjüngendem kreisförmigen bzw. runden Querschnitt (Hohlkegelkapillare) Flüssigkeit zu fördert. Aufgrund der Kontinuität ergibt sich ein konstanter Volumenstrom längs der Kapillare, der dann Schergeschwindigkeiten erzeugt, die sich längs der Kapillare stetig ändern (ansteigen). Über die Druckmeßwertgeber P1, P2 und P3 können dann die Druckabfälle $\Delta p_1$ und $\Delta p_2$ längs zwei Abschnitten A1 und A2 ermittelt werden, aus denen mit den entsprechenden bekannten Rechenalgorithmen, z. B. A. Pabedinskas, W. R. Cluett, S. T. Balke in Polymer Engineering and Sciene, Mid.March 1991, Vol. 31, No. 5, Seiten 365-375, die Fließexponenten m oder n und die entsprechenden Viskositäten errechnet werden können, z. B. mittels eines angeschlossenen (Prozeß—) Rechners. Es kann dazu der Druckabfall $\Delta p_1$ oder $\Delta p_2$ oder auch der Gesamtdruckabfall an den Druckmeßwertgebern zwischen P1 und P3 längs beider Abschnitte A1 plus A2 konstant gehalten werden. Eine Rückführpumpe 2 kann dann die Flüssigkeit in den Prozeß zurückfördern.

In Fig. 4 ist eine weitere Ausführungsform gezeigt, die der Ausführungsform in Fig. 3 entspricht, bei der die hohlkegelförmige Kapillare jedoch durch eine keilförmige Kapillare mit rechteckigem Querschnitt ersetzt wird. Der Keilkapillarenquerschnitt kann dabei längs der Achse sowohl in der Höhe H als auch der Breite B geändert werden. Die Betriebsweise entspricht der des Rheometers nach Fig. 3.

Fig. 5 beschreibt eine ähnlich vorteilhafte und erfindungsgemäße Ausführung mit zwei Kapillaren, die einen schlitzförmigen bzw. rechteckförmigen Querschnitt haben. Die Dosierpumpe 1 fördert die Flüssigkeit einer Schlitzkapillare S1 zu, die über eine gewisse Länge einen konstanten Querschnitt besitzt. Innerhalb dieses Bereiches mit konstantem Querschnitt wird der Druckabfall mit den Druckmeßwertgebern P1 und P2 ermittelt. Der ersten Schlitzkapillare S1 ist eine zweite Schlitzkapillare S2 nachgeordnet, die einen zur ersten Schlitzkapillare S1 unterschiedlich großen Querschnitt aufweist, der über eine gewisse Länge konstant bleibt, über die mit den Druckmeßwertgebern P3 und P4 der Druckabfall gemessen wird. Die Querschnitte sollen ein solches Verhältnis haben, daß sich die entsprechenden Schergeschwindigkeiten um den Faktor 2 bis zum Faktor 25 unterscheiden. In vorteilhaften Ausführungen können Abmessungen dabei die Schlitzhöhe H von 0,2 bis 12 mm und die Schlitzbreiten B von 4 mm bis 25 mm betragen. Mit den zwischen den Druckmeßwertgebern P1 und P2 und den Druckmeßwertgebern P3 und P4 ermittelten Druckabfällen $\Delta p_1$ und $\Delta p_2$ und den Schergeschwindigkeiten $\dot\gamma_1$ und $\dot\gamma_2$ in den Schlitzkapillaren S1 und S2 ist dann mit Gl. (5) der Fließexponent berechenbar. Mit dem Fließexponenten und den Schergeschwindigkeiten $\dot\gamma_1$ und $\dot\gamma_2$ kann dann die Fließfunktion als Ostwald-de Waele Flüssigkeit dargestellt werden.

Fig. 6 zeigt ein Rheometer mit zwei Meßkapillaren mit kreisförmigem konstantem Querschnitt. Die Dosierpumpe 1 fördert die zu testende Flüssigkeit durch eine erste Kapillare K1. Vor und hinter der ersten Kapillare K1 wird der Druck mit einem Druckmeßwertgeber P1 bzw. P2 gemessen. Derselbe Volumenstrom fließt dann durch eine zweite Kapillare K2 mit konstantem, aber kleinerem Querschnitt. Hier wird der Druckabfall durch die Druckmeßwertgeber P2 vor und P3 hinter der Kapillare K2 ermittelt. Die Kapillaren K1 und K2 haben unterschiedlich große Querschnitte, wobei diese unterschiedlich großen Querschnitte vorteilhaft so gewählt werden, daß sich bei gleichem Volumenstrom die Schergeschwindigkeit um den Faktor 2 bis um den Faktor 25 unterscheiden. Die Durchmesser können dabei üblicherweise von 0,2 mm bis 8 mm gewählt werden. Der beispielhaften Ausführung in Fig. 6 ist eine zweite Pumpe 2 als Rücklaufpumpe nachgeschaltet, die die getestete Flüssigkeit in einen Prozeß unter Druck zurückfördern kann. (Diese Ausführung kann zur Reduzierung der Ansprechzeit auch in einen zusätzlichen Pumpenkreislauf eingebaut werden, der die zu testende Flüssigkeiten schnell und unabhängig von dem momentanen Volumenstrom in der Meßanordnung zur Dosierpumpe 1 fördert). Diese Anordnung wird erfindungsgemäß so betrieben, daß die Drehzahl der Dosierpumpe 1 so geregelt wird, daß entweder die Druckdifferenz p zwischen den Druckmeßwertgebern P1 und P2 oder zwischen den Druckmeßwertgebern P2 und P3 oder die Gesamtdruckdifferenz zwischen P1 und P3 konstant gehalten wird.

In den in den Fig. 3 bis 6 dargestellten Rheometern geben Strömungspfeile eine Strömung von den großen Kapillarquerschnitten zu den kleinen Kapillarquerschnitten an. Damit wird die zu testende Flüssigkeit zuerst niedrigen und dann höheren Schergeschwindigkeiten unterworfen. Grundsätzlich können die Vorrichtungen auch in umgekehrter Richtung betrieben werden, so daß die Flüssigkeit zunächst die engen und dann die weiteren Querschnitte passiert und die Schergeschwindigkeit in der Flüssigkeit zunächst hoch ist und dann abfällt. Für alle dargestellten Rheometer wäre dann die Pumpe 2 die Dosierpumpe und die Pumpe 1 die Rückführpumpe. Am Ende der Kapillarenanordnung kann die Flüssigkeit auch ins Freie abgeführt werden.

Tabelle

| PMMA | | Mode I: $\dot{\gamma}_1 = 10\ s^{-1}$ | | | | |
|---|---|---|---|---|---|
| | $\eta_O$/Pas | $m(\dot{\gamma}_1)$ | $n(\dot{\gamma}_1)$ | $\eta(\dot{\gamma})$/Pas | $\eta_O/\eta(\dot{\gamma}_1)$ |
| 8H | $7,6 \cdot 10^4$ | -0,60 | 0,40 | $1,60 \cdot 10^4$ | 4,65 |
| 7N | $7,8 \cdot 10^3$ | -0,32 | 0,68 | $5,00 \cdot 10^3$ | 1,55 |
| 5N | $2,0 \cdot 10^3$ | -0,15 | 0,85 | $1,80 \cdot 10^3$ | 1,10 |
| 1 | 2 | 3 | 4 | 5 | 6 |
| PMMA | | Mode II: $\tau_1 = 5 \cdot 10^4$ Pa | | | |
| | $\eta_O$/Pas | $m(\tau_1)$ | $n(\tau_1)$ | $\eta(\tau_1)$/Pas | $\eta_O/\eta(\tau_1)$ |
| 8H | $7,6 \cdot 10^4$ | -0,32 | 0,68 | $4,75 \cdot 10^4$ | 1,60 |
| 7N | $7,8 \cdot 10^3$ | -0,32 | 0,68 | $4,90 \cdot 10^3$ | 1,59 |
| 5N | $2,0 \cdot 10^3$ | -0,32 | 0,68 | $1,25 \cdot 10^3$ | 1,60 |
| 1 | 2 | 7 | 8 | 9 | 10 |

Tabelle

Fließkomponenten m ($\dot{\gamma}_1$) der Viskositätsfunktion bzw. n ($\dot{\gamma}_1$) der Schubspannungsfunktion bei konstanter Scherge-schwindigkeit $\dot{\gamma}_1$ (Mode I) und Fließexponenten m($\tau_1$) der Viskositätsfunktion bzw. n($\tau_1$) der Schubspannungsfunk-tion bei konstanter Schubspannung $\tau_1$ (Mode II). 8H, 7N, 5N sind drei verschiedene jedoch viskos ähnliche Acrylglassorten. Die Viskosität bei der Schergeschwindigkeit $\dot{\gamma}_1$ ist $\dot{\gamma}(_1)$ und die Viskosität bei der Schubspannung $\tau_1$ ist ($\tau_1$). $\eta_O$ ist die Grenzviskosität bei extrem kleinen Schergeschwindigkeiten $\dot{\gamma} — 0$.

**Patentansprüche**

1. Verfahren zur kontinuierlichen oder diskontinuierlichen Messung der Viskosität und deren Verteilung sowie des Schmelzindex MFI bzw. Volumenfließindex MVI von nicht-newtonschen, insbesondere strukturviskosen, viskos-ähnlichen und viskos-unähnlichen Flüssigkeiten, insbesondere von Polymerschmelzen, bei denen die Flüssigkeit mittels einer volumetrischen, regelbaren Dosierpumpe durch einen ersten Kapillarabschnitt und mindestens einen dem ersten Kapillarabschnitt in Serie nachgeschalteten weiteren Kapillarabschnitt geleitet wird, wobei die beiden Kapillarabschnitte unterschiedliche Querschnitte aufweisen, **dadurch gekennzeichnet,** daß der durch die Kapillarabschnitte geleitete Volumenstrom derart geregelt wird, daß der Druckabfall längs wenigstens zweier Kapillarabschnitte konstant gehalten wird, daß der von der Pumpe abgegebene Volumenstrom bestimmt wird, daß aus dem Volumenstrom unter Berücksichtigung der Geometrie der Kapillarabschnitte die Schergeschwindigkeiten $\dot{\gamma}_1$ und $\dot{\gamma}_2$ in den Kapillarabschnitten bestimmt werden, daß als Maß für die Strukturviskosität bzw. die Breite der Viskositätsverteilung der Flüssigkeit ein Fließexponent n bestimmt wird als

$$n = \frac{\log (\Delta p_1 / \Delta p_2)}{\log (\dot{\gamma}_1 / \dot{\gamma}_2)}\ ,$$

wobei $\Delta p_1$ und $\Delta p_2$ die Druckabfälle über den Kapillarabschnitten sind, und daß für jede Schergeschwindigkeit $\dot{\gamma}_1$, $\dot{\gamma}_2$ die Viskosität bestimmt wird als

$$\eta(\dot{\gamma}) = k\dot{\gamma}^{(n-1)} = k\dot{\gamma}^{m},$$

wobei k eine Konsistenz ist.

2. Verfahren nach Anspruch 1, dadurch gekennzeichnet, daß der Druckabfall über alle Kapillarabschnitte konstant gehalten wird.

3. Verfahren nach einem der Ansprüche 1 oder 2, dadurch gekennzeichnet, daß eine sich stetig verengende oder erweiternde Kapillare, insbesondere mit flach rechteckförmigem oder rundem Querschnitt, eingesetzt wird und über einen ersten und über einen weiteren Kapillarabschnitt jeweils der Druckabfall gemessen wird.

4. Verfahren nach einem der Ansprüche 1 oder 2, dadurch gekennzeichnet, daß die beiden Kapillarabschnitte durch einen sprungartigen oder stetigen Übergang miteinander verbunden sind und über beide Kapillarabschnitte jeweils der Druckabfall gemessen wird.

5. Verfahren nach einem der Ansprüche 1 oder 2, dadurch gekennzeichnet, daß zwei Kapillarabschnitte mit rundem Querschnitt eingesetzt werden und über jede der beiden Kapillarabschnitte der Druckabfall gemessen wird.

6. Verfahren nach einem der Ansprüche 1 bis 5, dadurch gekennzeichnet, daß der mittlere Querschnitt von zwei Kapillarabschnitten derart gewählt wird, daß das Verhältnis des mittleren Querschnitts im Bereich von 2 bis 25 liegt.

7. Kapillarrheometer zur kontinuierlichen oder diskontinuierlichen Messung der Viskosität und deren Verteilung sowie des Schmelzindex MFI bzw. Volumenfließindex MVI von nicht-newtonschen, insbesondere strukturviskosen, viskos-ähnlichen und viskos-unähnlichen Flüssigkeiten, insbesondere von Polymerschmelzen, mit einem ersten Kapillarabschnitt und mindestens einem weiteren, dem ersten Kapillarabschnitt in Serie nachgeschalteten Kapillarabschnitt, wobei die beiden Kapillarabschnitte unterschiedliche Querschnitte aufweisen, einer volumetrischen, regelbaren Dosierpumpe zur Zufuhr der Flüssigkeit zum ersten Kapillarabschnitt, Druckmeßgeräten (P1, P2, P3, P4), mit denen jeweils am Eingang und am Ausgang jedes Kapillarabschnitts der Druck bestimmbar ist, sowie einer Regeleinrichtung zur Einwirkung auf die Dosierpumpe,
**dadurch gekennzeichnet,** daß die Regeleinrichtung wenigstens den Druckabfall längs zweier Kapillarabschnitte auswertet und den durch die Kapillarabschnitte geleiteten Volumenstrom derart regelt, daß der Druckabfall längs wenigstens der beiden Kapillarabschnitte konstant gehalten wird, und daß der von der Pumpe abgegebene Volumenstrom mit einer Meßeinrichtung bestimmbar ist.

8. Kapillarrheometer nach Anspruch 7, dadurch gekennzeichnet, daß die Regeleinrichtung den Druckabfall längs aller Kapillarabschnitte auswertet.

9. Kapillarrheometer nach Anspruch 7, dadurch gekennzeichnet, daß eine sich stetig verengende oder erweiternde Kapillare vorgesehen ist und die Druckmeßeinrichtungen derart angeordnet sind, daß der Druckabfall über einen ersten Kapillarabschnitt und über weitere Kapillarabschnitte gemessen werden kann.

10. Kapillarrheometer nach Anspruch 9, dadurch gekennzeichnet, daß die Höhe und Breite der Kapillarquerschnitte konvergent oder divergent verlaufen.

11. Kapillarrheometer nach Anspruch 7, dadurch gekennzeichnet, daß die Kapillarabschnitte durch einen sich im Querschnitt vom Querschnitt des einen Kapillarabschnitts zum Querschnitt des anderen Kapillarabschnitts verändernden Übergang miteinander verbunden sind.

12. Kapillarrheometer nach Anspruch 7, dadurch gekennzeichnet, daß zwei Kapillarabschnitte mit rundem Querschnitt vorgesehen sind und über jedem der beiden Kapillarabschnitte der Druckabfall gemessen werden kann.

13. Kapillarrheometer nach einem der Ansprüche 7 bis 12, dadurch gekennzeichnet, daß das Verhältnis der mittleren Querschnitte von Kapillarabschnitten 2 bis 25 beträgt.

14. Kapillarrheometer nach einem der Ansprüche 7 bis 13, dadurch gekennzeichnet, daß dem Auslaß des letzten Kapillarabschnitts eine Rückführpumpe (2) nachgeschaltet ist.

**15.** Kapillarrheometer nach Anspruch 14, dadurch gekennzeichnet, daß die Rückführpumpe (2) durch einen gesonderten Regelkreis so regelbar ist, daß der Druck am Ausgang des letzten Kapillarabschnitts auf einen einstellbaren Wert konstant gehalten werden kann.

## Claims

**1.** Process for the continuous or discontinuous measurement of the viscosity and distribution thereof and of the melt flow index (MFI) and melt volume index (MVI) of non-Newtonian, in particular intrinsically viscous, viscous-like and non-viscous-like fluids, in particular of polymer melts in which the fluid is directed by means of a volumetric, controllable metering pump through a first capillary portion and at least one other capillary portion, which is connected in series to the first capillary portion, the two capillary portions having different cross-sections, characterised in that the volumetric rate of flow directed through the capillary portions is controlled such that the drop in pressure is kept constant across at least two capillary portions, that the volumetric rate of flow discharged by the pump is determined, that the shearing rates $\dot{\gamma}_1$ and $\dot{\gamma}_2$ in the capillary portions are determined from the volumetric rate of flow, taking into consideration the geometry of the capillary portions, that a flow exponent n is determined as the quantity for the intrinsic viscosity or the width of the viscosity distribution of the fluid as

$$n = \frac{\log(\Delta p_1 / \Delta p_2)}{\log(\dot{\gamma}_1 / \dot{\gamma}_2)},$$

wherein $\Delta p_1$ and $\Delta p_2$ are the drops in pressure across the capillary portions, and that the viscosity is determined for each shearing rate $\dot{\gamma}_1$ and $\dot{\gamma}_2$ as

$$\eta(\dot{\gamma}) = k\dot{\gamma}^{(n-1)} = k\dot{\gamma}^m,$$

wherein k is a consistency.

**2.** Process according to claim 1, characterised in that the drop in pressure is kept constant across all of the capillary portions.

**3.** Process according to either claim 1 or claim 2, characterised in that a constantly narrowing or widening capillary, in particular having a flat rectangular or round cross-section, is used and the drop in pressure is measured by means of a first and by means of one other capillary portion.

**4.** Process according to either claim 1 or claim 2, characterised in that the two capillary portions are connected to one another by a non-constant or constant transition and the drop in pressure is measured by means of both capillary portions.

**5.** Process according to either claim 1 or claim 2, characterised in that two capillary portions having a round cross-section are used and the drop in pressure is measured by means of each of the two capillary portions.

**6.** Process according to any one of claims 1 to 5, characterised in that the mean cross-section of two capillary portions is selected such that the ratio of the mean cross-section lies in the region of from 2 to 25.

**7.** Capillary rheometer for the continuous or discontinuous measurement of the viscosity and distribution thereof and of the melt flow index (MFI) and melt volume index (MVI) of non-Newtonian, in particular intrinsically viscous, viscous-like and non-viscous-like fluids, in particular of polymer melts, which capillary rheometer has a first capillary portion and at least one other capillary portion, which is connected in series to the first capillary portion, the two capillary portions having different cross-sections, and which has a volumetric, controllable metering pump for the supply of the fluid to the first capillary portion, pressure measurement devices (P1, P2, P3, P4), by means of which the pressure can be determined at the inlet and outlet of each capillary portion, and which has a control device which acts on the metering pump, characterised in that the control device evaluates at least the drop in pressure across two capillary portions and controls the volumetric rate of flow directed through the capillary portions such that the drop in pressure across at least the two capillary portions is kept constant, and in that the volumetric rate of flow discharged by the pump can be determined using a measurement device.

8. Capillary rheometer according to claim 7, characterised in that the control device evaluates the drop in pressure across all of the capillary portions.

9. Capillary rheometer according to claim 7, characterised in that a constantly narrowing or widening capillary is provided and the pressure measurement devices are arranged such that the drop in pressure can be measured by means of a first capillary portion and by means of further capillary portions.

10. Capillary rheometer according to claim 9, characterised in that the height and width of the capillary cross-sections extend in a convergent or divergent manner.

11. Capillary rheometer according to claim 7, characterised in that the capillary portions are connected to one another by a transition which changes in cross-section from the cross-section of the first capillary portion to the cross-section of the other capillary portion.

12. Capillary rheometer according to claim 7, characterised in that two capillary portions having a round cross-section are provided and the drop in pressure can be measured by means of each of the two capillary portions.

13. Capillary rheometer according to any one of claims 7 to 12, characterised in that the ratio of the mean cross-sections of capillary portions is from 2 to 25.

14. Capillary rheometer according to any one of claims 7 to 13, characterised in that a return pump (2) is connected to the outlet of the last capillary portion.

15. Capillary rheometer according to claim 14, characterised in that the return pump (2) can be controlled by a separate control circuit such that the pressure at the outlet of the last capillary portion can be kept constant at an adjustable value.

## Revendications

1. Procédé pour la mesure en continu ou en discontinu de la viscosité et de sa distribution, ainsi que de l'indice de fusion MFI ou de l'indice de fluidité volumique MVI de liquides non-newtoniens, en particulier de liquides structurellement visqueux, de liquides simili-visqueux et non simili-visqueux, en particulier de masses fondues de polymères, où le liquide est envoyé au moyen d'une pompe de dosage volumétrique réglable au travers d'un premier segment capillaire et d'au moins un autre segment capillaire branché en série après le premier segment capillaire, et où les deux segments capillaires sont de sections transversales différentes, caractérisé en ce que le débit volumique envoyé au travers des segments capillaires est réglé de telle manière que le gradient de pression est maintenu constant au long d'au moins deux segments capillaires, en ce que le débit volumétrique de la pompe est mesuré, en ce que les vitesses de cisaillement $\dot{\gamma}_1$ et $\dot{\gamma}_2$ dans les segments capillaires sont déterminées en fonction du débit volumétrique en prenant en compte la géométrie des segments capillaires, en ce que, comme mesure de la viscosité structurelle ou de la distribution de la viscosité du liquide, il est déterminé un exposant de fluidité n, avec

$$n = \frac{\log (\Delta p_1 / \Delta p_2)}{\log(\dot{\gamma}_1 / \dot{\gamma}_2)}$$

où $\Delta p_1$ et $\Delta p_2$ représentent les gradients de pression au droit des segments capillaires, et en ce que, pour chaque vitesse de cisaillement $\dot{\gamma}_1$, $\dot{\gamma}_2$, la viscosité est déterminée d'après la formule

$$\eta(\dot{\gamma}) = k\dot{\gamma}^{(n-1)} = k\dot{\gamma}^m,$$

où k est une consistance.

2. Procédé suivant la revendication 1, caractérisé en ce que le gradient de pression est maintenu constant au long de tous les segments capillaires.

3. Procédé suivant l'une ou l'autre des revendications 1 ou 2, caractérisé en ce qu'il est fait appel à un capillaire se rétrécissant ou s'élargissant de manière continue, avec notamment une section rectangulaire allongée ou circulaire, et que le gradient de pression est mesuré au droit d'un premier segment capillaire et d'un segment capillaire supplémentaire.

4. Procédé suivant l'une ou l'autre des revendications 1 ou 2, caractérisé en ce que les deux segments capillaires sont réunis par une zone où la transition d'un segment à l'autre se fait par saut brusque ou bien de manière continue et que le gradient de pression est mesuré au droit des deux segments capillaires.

5. Procédé suivant la revendication 1 ou la revendication 2, caractérisé en ce qu'il est fait appel à deux segments capillaires à section transversale circulaire et que le gradient de pression est mesuré au droit des deux segments capillaires.

6. Procédé suivant l'une quelconque des revendications 1 à 5, caractérisé en ce que la section transversale moyenne de deux segments capillaires est choisi de telle manière que le rapport des sections transversales moyennes se trouve à l'intérieur d' une fourchette allant de 2 à 25 .

7. Rhéomètre capillaire pour la mesure en continu ou en discontinu de la viscosité et de sa distribution ainsi que de l'indice de fusion MFI ou de l'indice de fluidité volumique MVI de liquides non-newtoniens, notamment les liquides structurellement visqueux, les liquides quasi-visqueux et non quasi-visqueux, en particulier de masses tondues de polymères, rhéomètre doté d'un premier segment capillaire et d'au moins un autre segment capillaire branché en série après le premier segment capillaire, et où les deux segments capillaires sont de sections transversales différentes, rhéomètre comprenant une pompe de dosage volumétrique réglable destinée à envoyer le liquide dans un premier segment capillaire, des appareils de mesure de la pression (P1, P2, P3, P4) au moyen desquels la pression peut être mesurée à l'entrée et à la sortie de chaque segment capillaire, ainsi qu'un dispositif de régulation agissant sur la pompe de dosage,
caractérisé en ce que le dispositif de régulation évalue le gradient de pression au droit de deux segments capillaires au moins et règle le courant volumétrique envoyé à travers les segments capillaires de telle manière que le gradient de pression est maintenu constant au moins au long des deux segments capillaires, et en ce que le débit volumétrique de la pompe est mesurable au moyen d'un dispositif de mesure.

8. Rhéomètre capillaire suivant la revendication 7, caractérisé en ce que le dispositif de régulation évalue le gradient de pression au long de tous les segments capillaires.

9. Rhéomètre capillaire suivant la revendication 7, caractérisé en ce qu'il est prévu un capillaire s'élargissant ou se rétrécissant de manière continue et que les dispositifs de mesure de la pression sont disposés de telle manière que peuvent être mesurés le gradient de pression au droit d'un premier segment capillaire et le gradient de pression au droit de segments capillaires supplémentaires.

10. Rhéomètre capillaire suivant la revendication 9, caractérisé en ce que les hauteurs et les largeurs des sections transversales des capillaires convergent ou divergent.

11. Rhéomètre capillaire suivant la revendication 7, caractérisé en ce que les segments capillaires sont réunis par une zone de transition où la section transversale passe progressivement de la section transversale d'un segment capillaire à la section transversale de l'autre segment capillaire.

12. Rhéomètre capillaire suivant la revendication 7, caractérisé en ce qu'il est prévu deux segments capillaires de section transversale circulaire et que le gradient de pression peut être mesuré au droit de chacun des deux segments capillaires.

13. Rhéomètre capillaire suivant l'une quelconque des revendications 7 à 12, caractérisé en ce que le rapport des sections transversales moyennes des segments capillaires se trouve dans une fourchette allant de 2 à 25.

14. Rhéomètre capillaire suivant l'une quelconque des revendications 7 à 13, caractérisé en ce que qu'une pompe de retour (2) est branchée en sortie du dernier segment capillaire.

15. Rhéomètre capillaire suivant la revendication 14, caractérisé en ce que la pompe de retour (2) peut être réglée au moyen d'un circuit de régulation séparé de telle manière que la pression à la sortie du dernier segment capillaire peut être maintenue constante à une valeur réglable.

Fig.1

Fig.2

Fig.3

Fig.4

Fig.5

Fig.6